# EUROPEAN PATENT APPLICATION

(11) **EP 1 985 322 A1**
(43) Date of publication of application: **29.10.2008**
(21) Application number: 08251522.2
(22) Date of filing: 25.04.2008
(51) Int. Cl.: A61M 5/142

(54) **Cellular-enabled medical monitoring an infusion system**

(30) Priority: 27.04.2007 US 914611 P
(71) Applicant: Animas Corporation, West Chester PA 19380 (US)
(72) Inventor: Butoi, Bogdan, PA 19333 (US); Getz, Steven, Malvern,PA 19355 (US); Natale, Joseph, Yardley, PA 19067 (US); O'Connor, Sean, West Chester (US); Qunlan, John, pa 19475 (US); Rechtiene, Michael, Strafferd,PA 19087 (US); Shipway, Ian, Bryn Mawr, PA 19010 (US)
(74) Representative: Tunstall, Christopher Stephen

(57) **Abstract**

Described is a medical infusion device that is configured to send and receive data on a real-time basis through the use of circuitry that enables the connection of the device to a cellular network. In one embodiment, a medical infusion device is provided that includes a housing having a drug reservoir, a first processing system and a drive mechanism adapted to receive and execute instructions from the first processing system. The drive mechanism is in mechanical cooperation with the drug reservoir for controlling expulsion of the drug from the reservoir. Medical infusion device also includes an input device for receiving external instructions and configured to transmit the external instructions to the first processing system, a display for receiving and displaying information from the first processing system, and a second processing system in communication with the first processing system for controlling communication between the first processing system and a remote processing system via a cellular network.

## Description

### FIELD OF THE INVENTION

The present invention relates to systems for medical monitoring and drug infusion devices, particularly blood glucose meters and insulin infusion pumps for use in diabetes care and treatment. Disclosed are medical devices for monitoring blood glucose levels and delivering insulin which may communicate with each other via a direct physical connection, radio frequency communication, infra red communication, or other means. Either or both devices may also include integrated circuitry or an add-on style appliance to permit the device to communicate remotely via wireless networks, and particularly cellular networks.

### BACKGROUND OF THE INVENTION

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia which, if left uncorrected, can lead to ketosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

In order to more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid which is then analyzed by the meter. In most cases, the meter has a display screen which shows the BG reading for the patient. The patient may then dose themselves with the appropriate amount, or bolus, of insulin. For many diabetics, this results in having to receive multiple daily injections of insulin. In many cases, these injections are self-administered.

Due to the debilitating effects that abnormal BG levels can have on patients, e.g., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed.

Conventional insulin pumps are worn on the patient's body, either above or below their clothing and are connected to a flexible infusion cannula via a length of tubing. These relatively small, unobtrusive devices typically store a quantity of insulin in a replaceable cartridge and include components to control operation of the pump such as a processing unit, a display screen, and input functions such as buttons or a keypad.

Another version of an insulin pump is referred to as a patch pump or micro pump. The patch pump is smaller than the conventional insulin pump and is worn directly on the skin, eliminating the tubing. Patch pumps typically store insulin in a replaceable cartridge or in an integrated drug reservoir. Operation of the patch pump is generally through a remote controller such as a personal digital assistant or a glucose meter. The remote controller of patch pumps and conventional insulin pumps may include the ability to run multiple insulin delivery programs, such as basal and bolus programs, to eliminate the need for injections of insulin via needles and syringes, by providing medication via an infusion device that can be worn by the patient for an extended period of time, usually in the range of 1-3 days.

Patients using conventional insulin pumps typically have the ability to program insulin delivery times and amounts into their pump's software, and enter their BG values into the pump via a data input system to deliver boluses of insulin in response to their activities, such as exercise and meal intake. Alternatively, the BG meter and pump may be in communication to permit the meter to transmit the BG reading to the pump along with a recommended bolus value, or to permit the pump or user to determine the appropriate bolus of insulin, if any. While the convenience of an insulin pump may improve the lifestyle of the patient and lessen the imposition of their disease on their normal activity, such persons are still susceptible to experiencing symptoms of diabetes which may render them unable to operate their meter, pump, or both, thereby leaving them unable to self-administer the necessary bolus of insulin in response to abnormal BG levels.

A need exists, therefore, for a system of BG monitoring and insulin delivery that may provide additional assistance to diabetics experiencing highly abnormal BG levels and are unable to self-administer an appropriate bolus of insulin. A need also exists to provide assistance to diabetics using insulin pumps, such as the elderly and young children, who may not be properly monitoring the BG levels, may not realize that their insulin reservoir is empty or expired, or are experiencing some other difficulty relating to their insulin monitoring and delivery devices. Finally, among other possible improvements to the freedom of lifestyle of diabetics, there exists a need for a BG monitoring and/or insulin delivery systems that provide greater and more valuable monitoring and control of a patient's condition by their healthcare provider, family, or others who may assist them in lessening the effects and imposition of their disease on their quality of life.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 is a simplified block diagram of a pump with integrated cellular technology according to an exemplary embodiment of the present invention;

FIG. 2 is a simplified block diagram of an add-on appliance removeably attached to an insulin pump according to an exemplary embodiment of the present invention;

FIG. 3 is a perspective view of an add-on appliance according to an exemplary embodiment of the present invention in which the add-on appliance is removeably attached to an insulin pump;

FIG. 4 is an exploded view of the add-on appliance and insulin pump shown in FIG. 3;

FIGS. 5 - 7 are perspective views of the add-on appliance shown in FIG. 3;

FIG. 8 is a perspective view of an add-on appliance according to another embodiment of the present invention in which the add-on appliance is removeably attached to an insulin pump; and

FIG. 9 is an exploded view of the add-on appliance and the insulin pump shown in FIG. 8.

### DETAILED DESCRIPTION OF ILLUSTRATIVE EMBODIMENTS OF THE INVENTION

Disclosed herein are illustrative embodiments of the invention and non-limiting examples of the application and use of such embodiments. The embodiments and examples are intended, whether specifically stated or not, to include reasonable variations and equivalents and no disclaimer of such variants or equivalents is intended unless explicitly stated.

Broadly stated, the inventive concept of the illustrative embodiments to be described relate to blood glucose (BG) monitoring and insulin infusion systems and methods that offer significant advantages over prior art methods by employing, without limitation, structure, electronic circuitry, and software/firmware that enable these devices to communicate with each other and with remote processing systems. In the various embodiments, it shall be described how the needs presented by limitations of prior art devices have been overcome by, for example, creating BG meters and insulin infusion pumps with the capability of sending and receiving data to remote systems via various wireless protocols such as GPRS on widely available networks, such as GSM networks. The data may be sent on-demand, at predefined times, in response to some external instruction (e.g., an SMS message received by the device from a healthcare provider) or as generated.

The device may also be programmed to recognize alert conditions, such as abnormal BG levels in the patient, expired or depleted insulin reservoirs, unusual drug delivery patterns, the user's failure to obtain their BG level in a timely manner, or other situation that might be of a concern to a healthcare provider, friend, family member, or other person with an interest or need to assist in the management of the patient's diabetes. The presently disclosed systems and methods provide users of these devices significant advantages through the ability of their devices to be in communication with remote systems, including without limitation, healthcare providers, data storage and analysis centers, emergency care providers, friends, family, and others who may have an interest or take an active role in the patient's condition or treatment.

Previously, some devices have been configured to permit data from insulin pumps and blood glucose meters to be downloaded to personal computers and from there uploaded to central servers. Such data transfers typically required cables and internet connections and caused a disruption in the patient's use of their device, as the device could normally not be used during the transfer process. Data could then be reviewed to determine the occurrence of past events. Such systems, however, were unable to treat the situation at a specific moment or to alert others of events in a real-time or near real-time manner. Issues relating to the patient's condition or the functioning of their treatment apparatus could only be evaluated on a historical basis. Further, the data being reviewed was static, making it generally unsuitable for use in improve the patient's treatment.

In contrast, the present devices, whether for BG monitoring, insulin infusion, or both, may be configured to send and receive data on a real-time basis, or near real-time basis, through the use, for example, of circuitry which enable the connection of one or both of the devices to a cellular network, such as GSM networks which are widely employed on an international basis for carrying telephone communications. Such a system permits the devices to send and receive data contemporaneously, or nearly so, with the occurrence of events, making the data much more powerful and useful to the patient, healthcare providers, emergency personnel, etc., by being able to become aware of and respond to the patient's condition and their hardware status on a substantially real-time basis. Moreover, the present system is capable of maintaining drug infusion functions concurrently with connectivity features.

Further, the presently disclosed systems and methods provide the ability to substantially improve data management relating to the diabetes care and treatment. By enabling the infusion pump or BG meter to actively and/or passively transmit data to a remote processing system, such as a central server, without requiring an interface, such as a wireless IP network, cellular phone, or other intermediary device, the patient's hardware achieves greater flexibility through the ability to share, send, and/or receive data without limitations imposed by the particular external interface hardware that the patient possesses (such as their cell phone, wireless network connection, etc.). Moreover, such apparatus and methods obviate changes in such technology, due to its lack of dependence on external interface hardware - in other words, the patient need not concern themselves with their choice of cell phone or service provider in order to retain functionality or compatibility with their diabetes management devices.

For patients such as the elderly or small children that may not have or typically carry a cell phone, this allows such a patient's healthcare provider, custodian, parent, guardian, physician, emergency service provider, or other to be able to receive data from the patient's medical devices. For example, a small child's device or devices may be programmed to send SMS text messages to a parent on a predetermined schedule with notifications of data relating to the child's insulin delivery. Such a message may include the most recent bolus information, the most recent BG level, the amount of insulin (or other drug) remaining, or any other data or combinations of data that can be collected or are maintained by one or both of the devices. The parent, a physician, or other person may also have the ability to send SMS text messages to the child's medical device(s), or other types of messages which may contain instructions to be processed or otherwise interpreted by the device or devices.

Such message may initiate an on-demand query for information, such as the historical BG levels or bolus deliveries, present operating parameters of the hardware, or other data. The messages may also provide the remote party the ability to initiate actions by the hardware, such as the delivery of a bolus. Such an action would typically be initiated by emergency personnel or a physician in instances where certain criteria are present that indicate that an immediate bolus is required. Such a situation may arise, for example, when a patient begins to experience hyperglycemia. In such a state, the patient may be unable to control their equipment to administer a bolus of insulin. Having the ability to remotely detect such a condition by the appropriate personnel and to initiate action to remedy the condition is a great advantage to the patient, given that certain diabetes-related effects can be life-threatening or have profound long-term impact on the patient's health. An additional aspect of the use of cellular technology may be the inclusion of GPS positioning equipment within the medical device, to permit the immediate and accurate dispatch of emergency personnel to the user at the direction of the user or a remote party that is monitoring the patient's condition, thereby offering the ability to intervene in a critical situation much sooner than would be possible using prior art diabetes management tools.

The same, or at least functionally equivalent, technology can be used to enable customer support systems to remotely troubleshoot medical devices, thereby reducing the number of devices that require replacement every year and to provide more efficient and reliable performance of the devices to their users. The system may provide diabetes management services, such as data upload, sharing, and analysis that can provide healthcare professionals and device manufacturers an enhanced ability to create devices and treatment programs that may improve the quality of life by diabetes sufferers.

Now, with greater detail regarding illustrative technical implementations of the various embodiments, the invention may be characterized illustratively and without limitation as an insulin pump, blood glucose meter, other medical device, or combinations thereof that are configured or adapted to include a data transmitter capable of establishing a connection to a GSM, or similar, network. The device would preferably use GSM and GSM-related technologies and protocols (e.g. SMS, GPRS, EDGE, UMTS), since GSM is the most prevalent international standard. The integrated pump may preferably have "quad-band" capability (850/900/1800/1900 MHz) to obtain the best international coverage. The integrated pump could alternately use different cellular technologies and protocols (e.g. CDMA, 1xRTT, EvDO) and related frequencies. The selection of technology, protocol and frequencies could depend upon geographical location, coverage, financial considerations and intended product features.

The cellular features enable the medical device to be connected to the internet via, for example, the GPRS network, anywhere in the world where GSM is available. The processing system in the infusion pump, BG meter, or other device may be adapted to include a GSM-capable microchip that can be inserted either inside the medical device as an embedded component, or outside the device (in the form of a separate device, clip-on attachment, or other form all of which may generally be referred to as an appliance). The appliance may connect to the medical device either though RF (Bluetooth, etc), IR, or other communication system. Although most cellular-enabled devices require service activation through a cellular service provider, the devices may be pre-programmed by the manufacturer for service previously arranged through a carrier, for the benefit and ease of the user. Such a system may also eliminate any technical issues relating to different cellular service providers.

Further, by having a predetermined service available to the pump or other medical device, the device can be configured to send data to a central server or any other location as preprogrammed by the manufacturer or determined and set later by the user or technician. Also, features such as messaging (SMS, email, etc) can be enabled though the same process. Although different manufacturers may choose to enable or disable various features common to cellular devices, one exemplary embodiment of the invention contemplates disabling voice communication through the medical device, thereby eliminating technical requirements associated therewith and to avoid excess battery drain required to operate the device as a phone. Limited voice communication which, for example, could only be initiated to or by emergency personnel or healthcare providers may also be implemented.

The previously described, or other embodiments, of the invention may employ the cellular connection to allow communication with the hardware by customer support personnel, to remotely troubleshoot hardware related issues, reprogram the device or devices, conduct diagnostic functions, etc. Device diagnostics and/or other information can be reviewed remotely by support personnel. Optionally, a customer support representative may remotely view the device's display screen, to enable the representative to provide more accurate and efficient assistance to the patient. The representative may also be able to assume control of the device remotely, for training, emergency, or other purposes.

In another illustrative embodiment of the invention, an insulin pump and BG meter are used in combination with a data transmitter that is operating on WI-FI and/or WI-MAX standards. This system may enable the medical devices to be connected to the internet via the wireless network anywhere in the world where WI-FI / WI -MAX is available. The means of connecting the devices to the area network may be employed in the pump or meter, depending on design choices, and communication between the two devices could be achieved via a variety of wired or wireless connection schemes. A WI-FI/WI-MAX enabling microchip can be either inserted inside the medical device as an embedded component or outside the device (in the form of a separate device, clip-on attachment, or other form all of which may generally be referred to as an appliance). The appliance may connect to the medical device either though RF (Bluetooth, etc), IR, or other communication system offering similar functionality.

In another illustrative embodiment of the invention, the BG meter is a continuous monitoring device (CGM), as compared to the episodic monitor. When communication technology is employed permitting either cellular or internet connectivity, the device may actively or passively send data from either the sensor or the controller of the CGM to a remote server or other destination (email, cell phone, etc) without any user intervention. This system would permit a person to monitor a patient's diabetes remotely, control their device or devices remotely, or perform real-time treatment and/or diagnosis without any physical contact with the device.

Further, an embodiment of the invention may use a BG meter containing any of the previous described communications devices and a connection with either of an infusion pump, a CGM system or any other medical and/or non-medical device. This system would allow data to pass passively through the connection to the remote processing system. This allows data originated by the infusion pump or other device to pass though the BG meter and be sent to a remote destination, server, cellular appliance, etc.

Employing any of the features previously described or combinations thereof, a central server may actively send messages back to the patient meter or infusion device relating to treatment goals, recommendations, reminders, etc., while also sending those messages to any other mobile device (cell phone, IP phone, PDA, laptop computer).

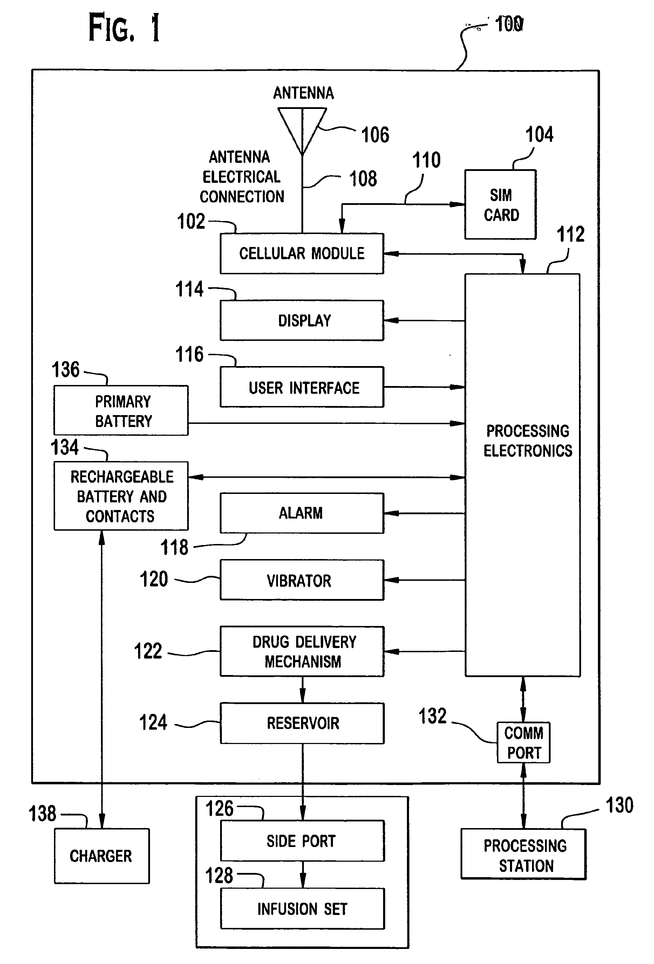

FIG. 1 illustrates a block diagram of an exemplary embodiment of the invention in which cellular technology is integrated into an insulin infusion pump 100. A cellular module 102, a GSM SIM card 104, an antenna 106, an antenna electrical connection 108 and an electrical connection 110 between the cellular module 102 and the SIM card 104 are provided within the pump housing and, preferably, as part of the processing electronics 112 that also drive a display 114, software for a user interface 116 and accepts data entry from a keypad or other data entry device. The processing electronics 112 also drive an alarm 118, a vibrator 120 and a drug delivery mechanism 122 for forcing a drug from a drug reservoir 124 (e.g., an insulin cartridge or insulin syringe) through a side port 126 connected to an infusion set 128 and into the body of the user. The processing electronics 112 may also include connectivity with, for example, a processing station 130 (e.g., a computer) through a variety of communications ports 132 such as USB, IR, RF, etc. In the configuration shown in FIG. 1, the pump housing holds the antenna 106 and electrical connections 108, 110 within the pump housing configured (e.g., enlarged) to fit the cellular module 102. In an alternative configuration, the pump housing may provide room for the antenna along a side of the pump. In yet another configuration, the cellular module and antenna could be miniaturized and integrated into the confines of an otherwise unmodified pump housing.

The integrated insulin pump 100 requires additional power for cellular communication, when compared with non-cellular enabled pumps. Several configurations are possible. In one configuration, a single rechargeable battery, e.g. a rechargeable lithium ion battery, may provide power to the integrated pump. In the embodiment shown in FIG. 1, separate power sources are provided for the cellular module 102 and the infusion pump functionality. The cellular module 102 is powered by a rechargeable battery 134 with a single primary battery 136 powering the infusion pump functionality. In a variation of this configuration, two rechargeable batteries may be used.

As shown in FIG. 1, the rechargeable battery 134 resides within the housing and a charger 138 is external to the housing. In another configuration, a chargeable battery is external to the pump. The external battery can be attached as part of a holster or as a sled that attaches to the pump. In the case of the sled, a seal around the connection point keeps the battery-connection contacts water resistant.

In configurations with internal rechargeable power, contacts on the pump housing may provide battery recharging. In an alternative configuration, a depleted rechargeable battery is swapped out with a charged battery. If the battery powers the entire integrated pump, high-power operations such as infusion deliveries might be suspended during the swap, and a bridge power source, such as a charged capacitor or coin-cell battery, could keep the pump otherwise operational.

It may be desirable to eliminate battery charging contacts and seals. In this case the battery(s) could be charged through low-frequency magnetic induction, using an induction coil within the integrated pump.

Power savings reduce the power consumption, thereby extending battery-recharge interval or, alternately, reducing required battery capacity and size. For example, the integrated pump can power up the cellular module only when needed to transmit data. If periodic communication is necessary, the integrated pump can power up the cellular module at an interval. For example, the module is powered-up every 15 minutes to check for waiting cellular messages, powered-off upon communication completion then powered-up again in 15 minutes. The interval of periodic communications could be automatically adjusted according to time of day and user input. For example, periodic checks for incoming cellular messages during the evening, when the patient is sleeping, may be unnecessary and reduced or eliminated. Further, it may be desirable to configure the integrated pump such that the cellular module is only powered when explicitly prompted by the user.

The user interface 116 on the insulin pump 100 could be used to convey cellular status and operating parameters. It could also be used as the means for user configuration, initiating or confirming cellular messages, and entering cellular phone numbers.

Complex pump configuration, such as entry of a telephone-number phone book, could be more readily accomplished with a PC and appropriate software. The PC would be equipped with the appropriate communications means and protocol (e.g. IR, RF, etc.) and it would communicate directly with the integrated pump.

### Exemplary GSM Implementation

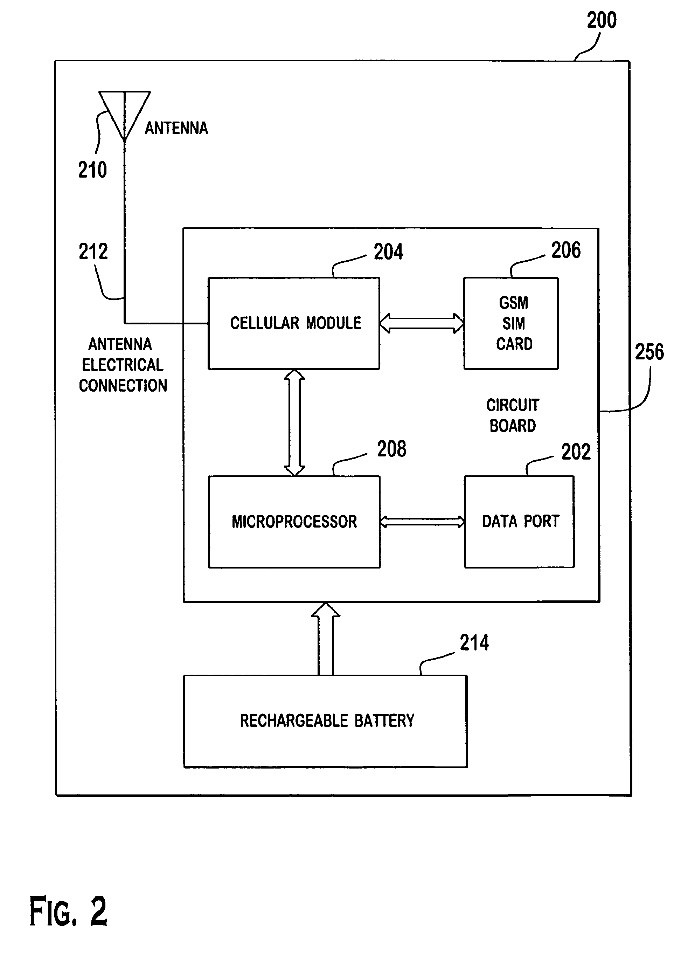

An infusion pump may include the following equipment, either embedded therein or in the form of an add-on appliance 200 (see FIG. 2) that interfaces with a pump via a data port 202, such as a USB port, IR port, RF antenna, or an equivalent data transfer connection apparatus existing on the pump and appliance in a corresponding or complementary manner:

Component list (non-exhaustive):

GSM cellular module 204 or chipset (the chipset may possibly be a single IC).

GSM SIM card 206

Microcontroller or microprocessor 208

Miscellaneous circuits, connectors and hardware

Antenna 210

Antenna electrical connection 212

The potential uses of this pump are as follows:

Parental control/monitoring

1) Parent calls child's pump and checks daily history and pump status.

2) Return message can be sent by email, web, cell phone text.

3) Parent sends text message to child's pump, e.g. "check BGs."

4) Message can be entered through cell phone or internet.

5) Pump beeps/vibrates and displays message.

6) Host sends text message to parent when critical activities occur.

7) Host sends email to parent with daily pump activities.

Record keeping

1) Transfer of pumper history for analysis at physician's office.

2) Periodic or transfer of pumper history to centralized database.

3) With proper attention to privacy concerns and disclosures, data may be available for mining of patterns and trends.

Pump training and troubleshooting

1) Monitoring of unusual pump activity, warnings, alarms.

For example, monitor new pumpers with Auto-off alarms. Alarms can be sent by email, cell phone text, or to phone (cell or POTS) through text-to-audio services.

2) Pair new pumpers, i.e., patients using insulin infusion pumps, with a mentor.

3) Pump settings and diagnostic routines could be accessed for troubleshooting.

4) Pump software and configuration could be remotely updated.

### Embodiment Employing Cellular Functionality via an Add-On Appliance

Cell-phone technology could be implemented in an insulin pump or BG Meter through an add-on appliance. The appliance could be self-contained and would not require hardware modifications to existing pumps or meters, although new devices may be created to have optional add-on attachments. The appliance could be housed in a plastic pump holster, leather pouch, clip-on module, or other device which may also be decorative. The appliance may be easily separated from the pump if necessary, e.g. for repairs or storage. Connectivity features and a processing system therefore may be housed within the attachably removable appliance housing.

The appliance may communicate with existing pumps or meters through an infrared (IR) transceiver, RF antenna, USB port, or other type of wireless or wired connection known in the art such as quasi-static fields (E-field, H-field), ultrasonics, or electrical contacts. If RF communications are used, multiple RF protocols are may be employed, e.g. Bluetooth, Zigbee, and Wibree.

The appliance may contain its own power source, e.g. a rechargeable lithium ion battery 214. Other implementations could use an alternate energy source, e.g. fuel cell. The battery could be recharged through an external charging device that would connect through electrical contacts or alternately through an induction coil. The charging control circuits could be contained in the charging mechanism or the appliance, or it could be distributed between the two devices.

To reduce appliance cost, power-consumption and complexity, standard cell phone features such as camera, speaker, display and keypad can be omitted. The appliance would instead have a minimal user interface (UI). For example it might contain two multicolor light emitting diodes (LED). The LEDs could convey simple operating parameters such as battery status, cellular connection status, etc. The information conveyed by the LEDs could be expanded by manipulating blinking rate, blinking duty cycle and LED color.

Because the appliance's user interface (UI) may have only limited functionality, the pump or meter's UI would be used to convey more complex appliance information. For example, a pump's display could be used to convey appliance text and graphical messages. The pump's keypad and menu structure could be used to confirm messages and to allow the user to input configuration information and commands.

Complex appliance configuration, such as entry of a telephone-number phone book, could be more readily accomplished with a personal computer (PC) and appropriate software. The pump and appliance could be separated for this operation. The PC would be equipped with the appropriate communications means and protocol (e.g. IR, RF, etc.) and it would communicate directly with the appliance.

The appliance would preferably use GSM and GSM-related technologies and protocols (e.g. SMS, GPRS, EDGE, UMTS), since GSM is the most prevalent international standard. The appliance would preferably have "quad-band" capability (850/900/1800/1900 MHz) to obtain the best international coverage. The appliance could alternately use different cellular technologies and protocols (e.g. CDMA, 1xRTT, EvDO) and related frequencies. The selection of technology, protocol and frequencies could depend upon geographical location, coverage, financial considerations and intended product features.

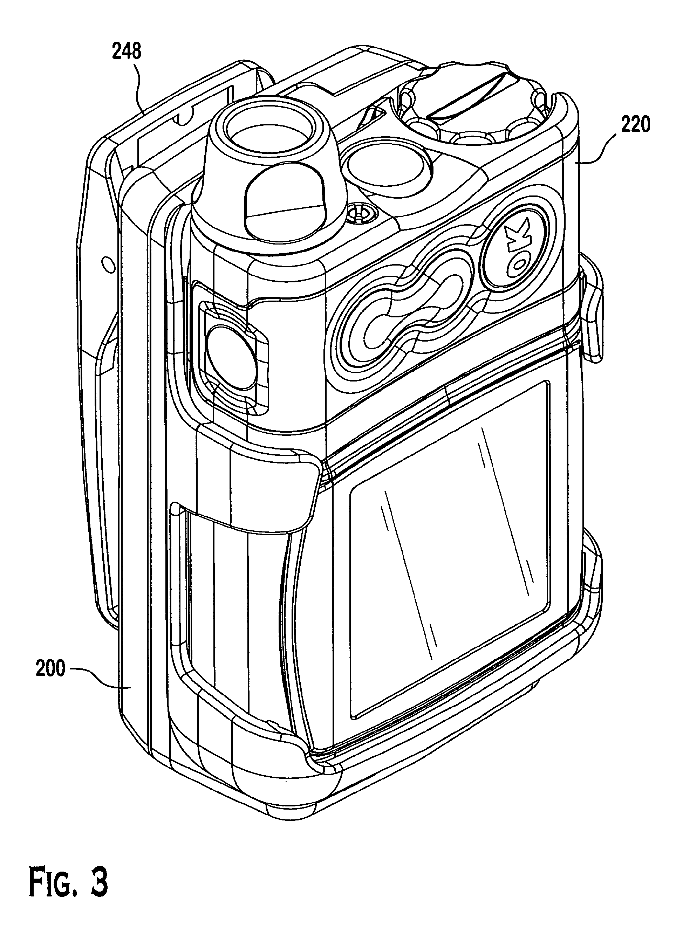

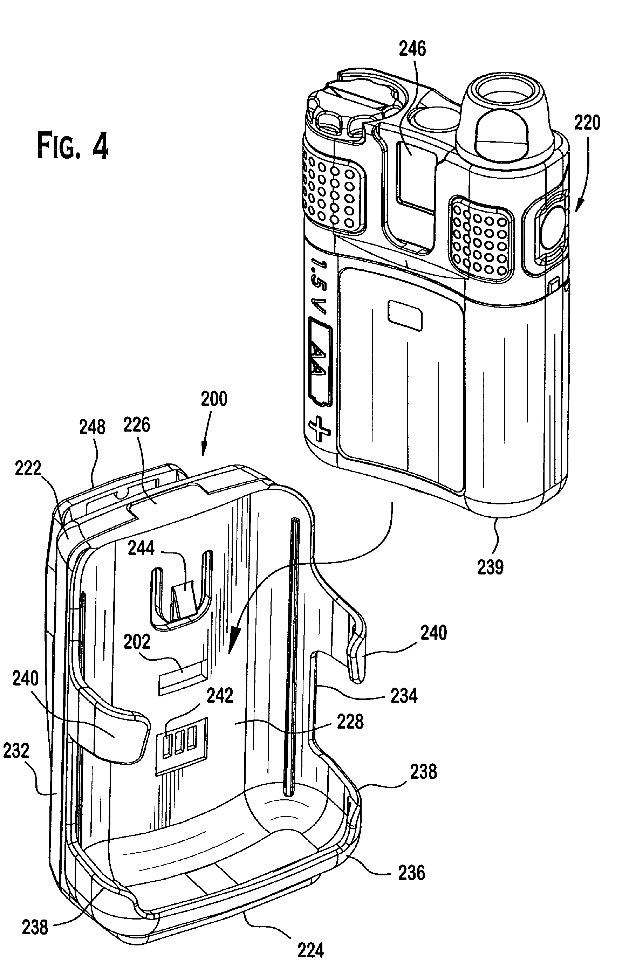

Referring to FIGS. 3 - 7, an exemplary embodiment of an add-on appliance for an insulin pump 220 is illustrated. The add-on appliance as shown in FIGS. 3-7 is a holster 200 that includes a housing 222 having a first end 224, a second end 226, a first surface 228, a second surface 230, a first side 232 and a second side 234. A cradle portion 236 protrudes from the first surface 228 of the housing 222 at the first end 224. The cradle portion 236 is concave in shape with at least one side 238 curved upward toward the second end 226 of the housing 222 such that when the insulin pump 220 is engaged with the cradle portion 236, the cradle portion 236 cradles an end 239 of the pump 220. At least one arm 240 protrudes from the first surface 228 at either the first side 232 or the second side 234 of the housing 222. In the embodiment shown in FIGS. 3-7, two arms 240 protrude from and curve inward toward the first surface 228. An arm 240 is located at about the middle of each of the first and second sides 232, 234 of the housing 222. The cradle portion 236 and the at least one arm 240 are intended to releasably engage the insulin pump 220 when the insulin pump 220 is inserted into the holster 200.

The first surface 228 of the housing 222 further includes at least one opening 242 through which battery charger contacts are accessible, a data port 202 (e.g., an IR port), and an optional spring catch 244 that releasably engages a recess 246 in the housing of the insulin pump 220 when the pump 220 is inserted into the holster 200.

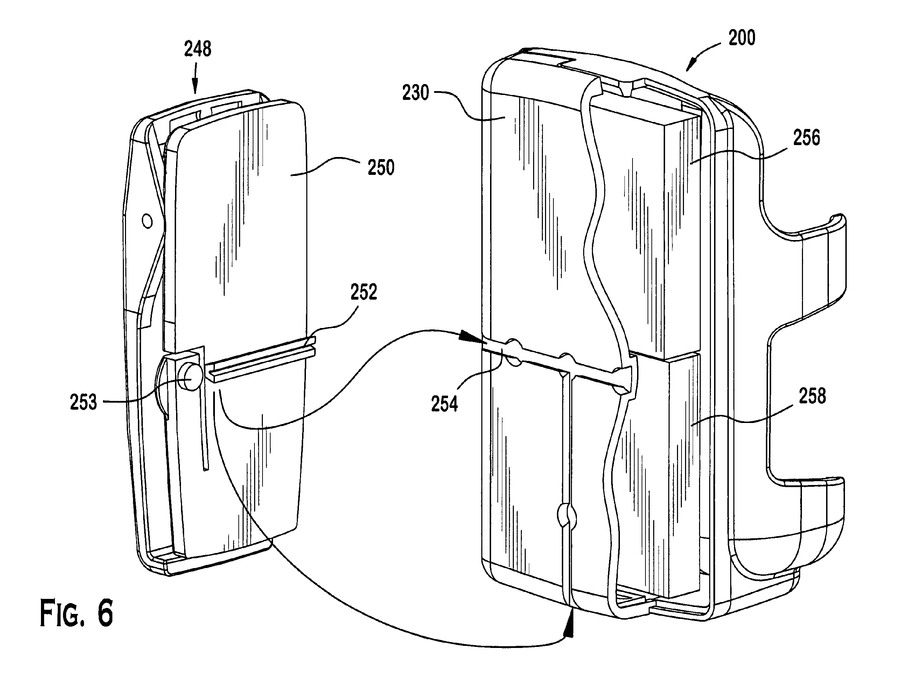

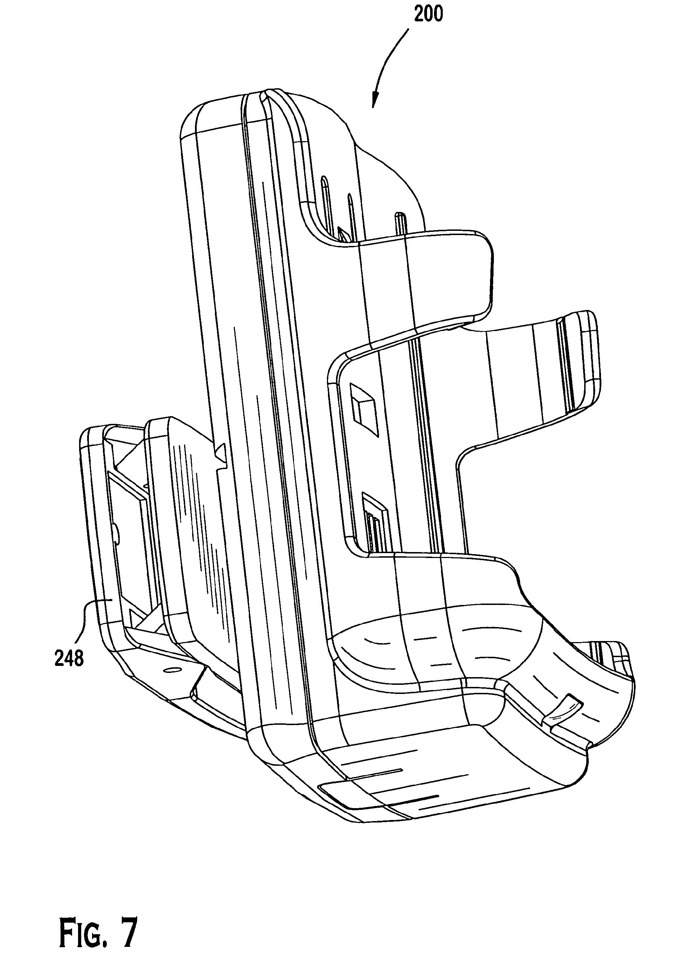

As shown in FIG. 6, the second surface 230 of the holster housing 220 may include a clip 248 that may be removably attached to, for example, a belt of the user. A surface 250 of the clip 248 includes a rail 252 in line with a pivot 253, both of which mate with and move within a T-shaped slot 254 on the second surface 230 of the holster 200. The rail 252 and pivot 253 on the clip 248 and the slot 254 on the holster 200 allow the user to removably attach the clip 248 to the holster 200 in various positions such as, for example, in a vertical or a horizontal orientation (see FIGS. 6 and 7, respectively).

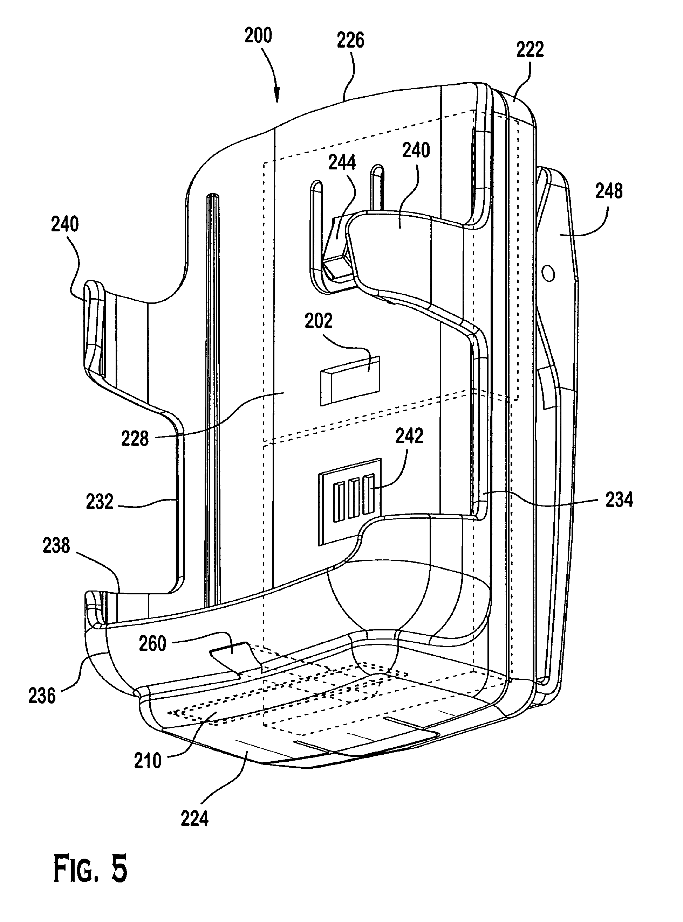

Referring again to FIG. 6, the holster 200 further includes a circuit board 256 having a GSM cellular module or chipset (the chipset may possibly be a single IC), a SIM card, a SIM connector or optional holder, a microprocessor, microprocessor circuits, an IR transceiver, and an antenna connector. Referring to FIGS. 5 and 6, also included in the holster 200 are an antenna 210 connected to the antenna connector 212, a rechargeable battery 258, for example a lithium-ion or lithium-polymer battery, battery charger contacts and an optional LED light pipe 260 that conveys simple operating parameters such as battery status or cellular connection status. As shown in FTG. 5, the antenna 210 may be located inside the first end 224 of the housing 222 underneath the cradle portion 236. The antenna 210 may alternatively be located anywhere within the housing 222 where space allows and as long as the signal transmitted and received is not blocked.

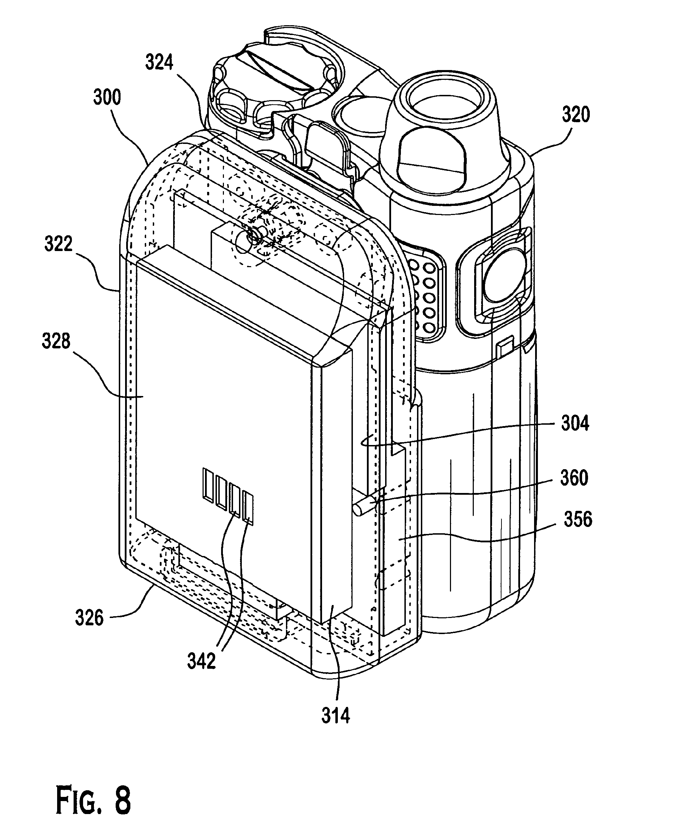

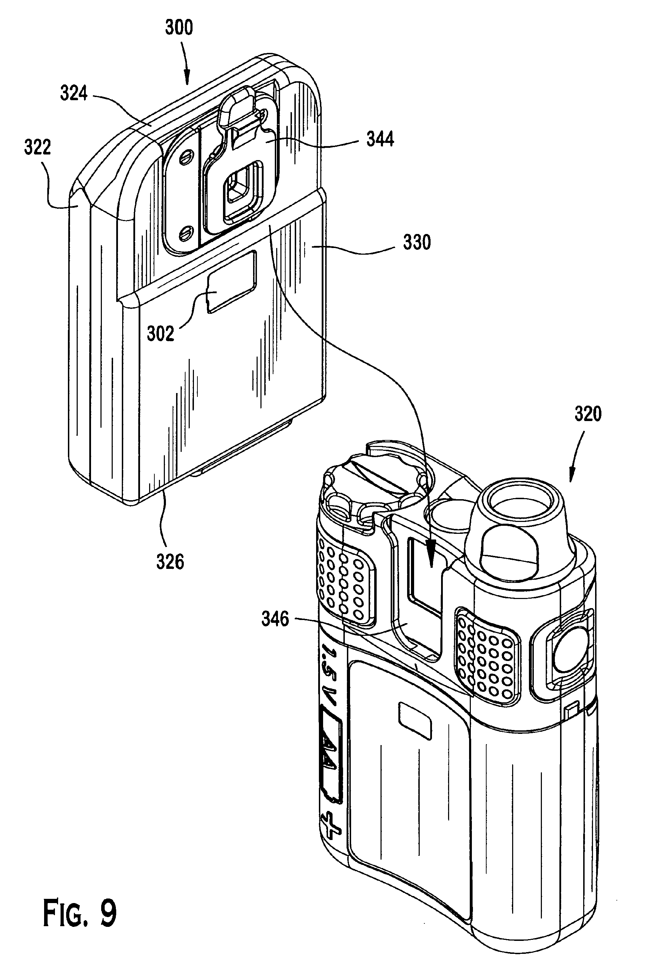

FIGS. 8 - 9 illustrate another exemplary embodiment of an add-on appliance 300 for an insulin pump 320 in which the add-on appliance 300 houses cellular functionality. The add-on appliance 300 includes a housing 322 having a first end 324, a second end 326, a first surface 328 and a second surface 330. The first surface 328 of the housing 322 further includes at least one opening 342 through which battery charger contacts are accessible. The second surface 330 includes a data port 302 (e.g., an IR port), and a latch 344 that releasably engages a recess 346 in the housing of the insulin pump 320 when the add-on appliance 300 is engaged with the pump 320.

The add-on appliance 300 further includes a circuit board 356 with a GSM cellular module 304 or chipset (the chipset may possibly be a single IC) and a GSM SIM card, a GSM SIM connector or optional holder, a rechargeable battery 314 such as a lithium-ion or a lithium-polymer battery, an IR transceiver, a microcontroller or microprocessor, an antenna and one or more LEDs 360 that convey simple operating parameters such as battery status or cellular connection status. The antenna may be located anywhere within the housing 322 where space allows and as long as the signal transmitted and received is not blocked.

### ELEMENTS

| | |
|---|---|
| 100 | Infusion pump |
| 102 | Cellular module |
| 104 | SIM card |
| 106 | Antenna |
| 108 | Antenna electrical connection |
| 110 | Electrical connection |
| 112 | Processing electronics |
| 114 | Display |
| 116 | User interface |
| 118 | Alarm |
| 120 | Vibrator |
| 122 | Drug delivery mechanism |
| 124 | Drug reservoir |
| 126 | Side port |
| 128 | Infusion set |
| 130 | Processing station |
| 132 | Communication port |
| 134 | Rechargeable battery |
| 136 | Primary battery |
| 138 | charger |
| 200 | Holster type add-on appliance |
| 202 | Data port |
| 204 | Cellular module |
| 206 | SIM card |
| 208 | Microprocessor |
| 210 | Antenna |
| 212 | Antenna electrical connection |
| 214 | Rechargeable battery |
| 220 | Pump |
| 222 | Housing |
| 224 | First end |
| 226 | Second end |
| 228 | First surface |
| 230 | Second surface |
| 232 | First side |
| 234 | Second side |
| 236 | Cradle portion |
| 238 | Cradle side |
| 239 | Pump end |
| 240 | Arm |
| 242 | Opening |
| 244 | Spring catch |
| 246 | Recess |
| 248 | Clip |
| 250 | Surface of the clip |
| 252 | Rail |
| 253 | Pivot |
| 254 | Slot |
| 256 | Circuit board |
| 258 | Battery |
| 260 | Light pipe |
| 300 | Add-on appliance |
| 302 | Data port |
| 304 | Cellular module |
| 314 | battery |
| 320 | Pump |
| 322 | Housing |
| 324 | First end |
| 326 | Second end |
| 328 | First surface |
| 330 | Second surface |
| 342 | Openings |
| 346 | Recess |
| 356 | Circuit board |
| 360 | LED |

## Claims

1. A medical infusion device comprising:
a housing having a drug reservoir;
a first processing system located within the housing;
a drive mechanism within the housing and adapted to receive and execute instructions from the first processing system, wherein the drive mechanism is in mechanical cooperation with the drug reservoir for controlling expulsion of the drug from the reservoir;
an input device for receiving external instructions and configured to transmit the external instructions to the first processing system;
a display for receiving and displaying information from the first processing system; and
a second processing system in communication with the first processing system for controlling communication between the first processing system and a remote processing system via a cellular network.

2. The medical infusion device of claim 1, wherein the second processing system is disposed within the pump housing.

3. The medical infusion device of claim 1, wherein the second processing system is disposed in a second housing.

4. The medical infusion device of claim 3, wherein the second housing includes a first communications port and the pump housing includes a second communications port.

5. The medical infusion device of claim 4, wherein the second housing is configured to be removably engaged with the pump housing in a manner permitting the first communications port to communicate with the second communications port.

6. The medical infusion device of claim 5, wherein the second housing is a holster-type housing having a cradle portion and at least one arm that removably engage the pump housing.

7. The medical infusion device of claim 3, wherein the first processing system and second processing system communicate via a wireless connection.

8. The medical infusion device of claim 7, wherein the wireless connection is selected from the group consisting of an RF connection and an IR connection.

9. The medical infusion device of claim 3 wherein the first processing system and second processing system communicate via a wired connection.

10. The medical infusion device of claims 2 or 3, wherein the second processing system communicates with the remote processing system via a general packet radio service (GPRS) connection.

11. The medical infusion device of claim 10, wherein the second processing system is capable of establishing a connection with a cellular network.

12. The medical infusion device of claim 11, wherein the cellular network is a GSM network.
